# EUROPEAN PATENT APPLICATION

(11) **EP 4 785 997 A1**
(43) Date of publication of application: **05.08.2026**
(21) Application number: 23953413.4
(22) Date of filing: 26.09.2023
(51) Int. Cl.: A61N 7/00

(54) **ABLATION CATHETER AND ABLATION DEVICE**

(71) Applicant: Shenzhen Pulsecare Medical Technology Co., Ltd., Shenzhen, Guangdong 518048 (CN)
(72) Inventor: TAN, Jianwen, Shenzhen, Guangdong 518000 (CN)
(74) Representative: Metida
(86) International application number: PCT/CN2023/121611
(87) International publication number: WO 2025/065253

(57) **Abstract**

The present application is applicable to the technical field of medical devices, and provides an ablation catheter (100) and an ablation device. The ablation catheter (100) includes a catheter assembly (10) and an ultrasonic generator (20) arranged in the catheter assembly, wherein the ultrasonic generator includes a driving assembly (21) and a generation assembly (22) connected to the driving assembly, the driving assembly is configured for driving the generation assembly to vibrate, the generation assembly is configured for emitting ultrasonic waves and converging the ultrasonic waves to a focus, and the focus is located out of the catheter assembly. The ablation device includes the above ablation catheter. According to the ablation catheter provided by the present application, the ultrasonic waves emitted by the generation assembly can be converged to form a focus outside a blood vessel wall, so that the ultrasonic waves can have larger energy at the focus to ablate sympathetic nerves at a position corresponding to the focus. Further, at positions where the ultrasonic waves pass through the blood vessel wall, the ultrasonic waves are dispersed and carry less energy, resulting in less heat generating of the blood vessel wall, thereby alleviating the damage caused by the ultrasonic waves to the blood vessel wall.

## Description

### TECHNICAL FIELD

The present application relates to the technical field of medical devices, and in particular to an ablation catheter and ablation device.

### BACKGROUND

Renal Denervation (RDN) refers to the use of interventional treatment methods (such as via the femoral or radial arteries) to destroy the sympathetic afferent and efferent nerves of the kidney, weaken the sympathetic nerve activity of the kidney and throughout the body, thereby lowering blood pressure.

RDN technology may be based on manners such as radiofrequency ablation, ultrasound ablation, cryoablation, chemical ablation, etc, but currently, radiofrequency ablation (rRDN) and ultrasound ablation (uRDN) are mainly dominant. Among them, ultrasound ablation has the advantages of good penetration and high energy controllability, and thus has certain technical advantages in the field of RDN.

At present, ultrasound ablation mainly adopts two schemes: circular transducers with 360° energy emission and planar transducers with directional energy emission. However, whether the circular transducers or the planar transducers, their ultrasound energy distribution cannot achieve protection of the renal artery vessel wall, and other cooling methods must be adopted to achieve protection of the renal artery vessel, which increases the risk of renal artery injury and stenosis.

### SUMMARY

An object of the present application is to provide an ablation catheter and an ablation device that can alleviate the negative impact of ultrasonic waves on the renal artery vessel wall during the ablation of sympathetic nerves of the renal artery.

On one aspect, embodiments of the present application provide an ablation catheter, including:
a catheter assembly; and
an ultrasonic generator arranged in the catheter assembly, wherein the ultrasonic generator includes a driving assembly and a generation assembly connected to the driving assembly, the driving assembly is configured for driving the generation assembly to vibrate, and the generation assembly is configured for emitting ultrasonic waves and converging the ultrasonic waves to a focus, wherein the focus is located out of the catheter assembly.

In some embodiments, the generation assembly includes an arc-shaped piezoelectric sheet, wherein a circle center of the piezoelectric sheet and the focus are disposed on the same side of the catheter assembly.

In some embodiments, a radius of the piezoelectric sheet is in a range of 6mm to 15mm.

In some embodiments, at least one separating groove is defined in the piezoelectric sheet to divide the piezoelectric sheet into at least two piezoelectric sub-sheets, and the piezoelectric sub-sheets are rotatable relative to the driving assembly.

In some embodiments, the generation assembly operates in a first state or a second state, and an acoustic power of the ultrasonic waves emitted by the generation assembly in the second state is less than or equal to 10% of that in the first state.

In some embodiments, the ablation catheter further includes an imaging device arranged in the catheter assembly and located at a lateral side of the ultrasonic generator.

In some embodiments, the catheter assembly includes an outer catheter and a rotary member arranged in the outer catheter, the outer catheter is provided with a receiving chamber, and the ultrasonic generator is accommodated in the receiving chamber; and
one end of the rotary member extends into the receiving chamber and is connected to the ultrasonic generator, and the rotary member is bendable along with the outer catheter and rotatable relative to the outer catheter to drive the ultrasonic generator to rotate.

In some embodiments, the catheter assembly further includes an inlet channel and an outlet channel provided on the outer catheter, and one end of the inlet channel and one end of the outlet channel both are communicated with the receiving chamber.

In some embodiments, the catheter assembly further includes a covering film arranged around the outer catheter, the covering film encircles a covering cavity around an outer periphery of the outer catheter, the covering cavity is communicated with the receiving chamber, and the covering film is movable in a direction towards or away from the receiving chamber.

In some embodiments, the catheter assembly further includes a support member and a moving member arranged out of the outer catheter, one end of the support member is connected to the outer catheter, and another end of the support member is connected to the moving member; and
the moving member is connected to the outer catheter in a movable manner, the moving member is movable relative to the outer catheter along an axial direction of the outer catheter, and is able to drive the support member to deform in a direction away from or close to the outer catheter.

In some embodiments, an opening is defined in the outer catheter and communicates with the receiving chamber, and the opening is oriented toward the ultrasonic generator; and
a transaudient film is provided on the opening, wherein the transaudient film is configured for sealing the opening and allowing the ultrasonic waves to pass through.

In some embodiments, the ablation catheter further includes a temperature sensor arranged at a lateral side of the ultrasonic generator.

On another aspect, embodiments of the present application provide an ablation device including the above ablation catheter.

Compared with the prior art, embodiments of the present application have the following beneficial effects: the ultrasonic generator is configured with the driving assembly and the generation assembly, and the ultrasonic waves emitted by the generation assembly can be configured to form a focus outside a blood vessel wall, so that the ultrasonic waves can have larger energy at the focus to ablate sympathetic nerves at a position corresponding to the focus. Further, at positions where the ultrasonic waves pass through the blood vessel wall, the ultrasonic waves are dispersed and carry less energy, resulting in less heat generating of the blood vessel wall, thus alleviating the damage caused by the ultrasonic waves to the blood vessel wall.

### BRIEF DESCRIPTION OF THE DRAWINGS

In order to illustrate the technical solution in embodiments of the present application more clearly, the following briefly introduces accompanying drawings used in the description of the embodiments. Obviously, the accompanying drawings in the following description are only some embodiments of the present application. Those of ordinary skill in the art can obtain other accompanying drawings from these accompanying drawings without any creative efforts.
FIG. 1 is a partial three-dimensional view of an ablation catheter provided by an embodiment of the present application.
FIG. 2 is a partial three-dimensional view of an ablation catheter provided by an embodiment of the present application.
FIG. 3 is a cross-sectional view of the ablation catheter of FIG. 1.
FIG. 4 is an enlarged view of part A of FIG. 3.
FIG. 5 is a cross-sectional view taken along line B-B of FIG. 3.
FIG. 6 is a cross-sectional view of the ablation catheter of FIG. 2.
FIG. 7 is an enlarged view of part C of FIG. 6.
FIG. 8 is a cross-sectional view taken along line D-D of FIG. 6.
FIG. 9 is a three-dimensional view of an ultrasonic generator of an ablation catheter provided by an embodiment of the present application.
FIG. 10 is a schematic view of a piezoelectric sheet of an ablation catheter provided by an embodiment of the present application.

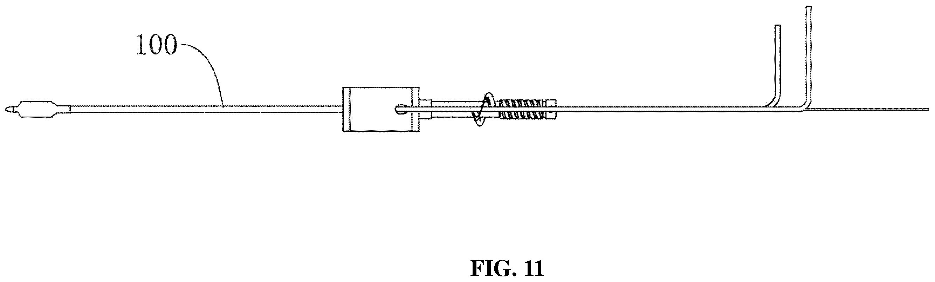

FIG. 11 is a three-dimensional view of an ablation device provided by an embodiment of the present application.

### Reference numbers in the drawings:

100, ablation catheter;
10, catheter assembly; 11, outer catheter; 111, receiving chamber; 112, opening; 113, end fitting; 12, rotary member; 13, inlet channel; 14, outlet channel; 141, water pipe; 15, covering film; 151. covering cavity; 16, support member; 17, moving member; 18, transaudient film;
20, ultrasonic generator; 21, driving assembly; 22, generation assembly; 221, piezoelectric sheet; 2211, separating groove; 2212, piezoelectric sub-sheet; 23, cable;
30, imaging device; and
40, temperature sensor.

### DESCRIPTION OF THE EMBODIMENTS

In order to make the purpose, technical solution, and advantages of this application clearer and more understandable, the following will provide further detailed description of this application in combination with the accompanying drawings and embodiments. It should be understood that the specific embodiments described herein are only used to explain the present application and are not intended to limit the present application.

It should be noted that when a component is referred to as "fixed to" or "set to" another component, it can be directly or indirectly attached to another component. When a component is referred to as "connected to" another component, it can be directly or indirectly connected to another component. The terms "up", "down", "left", "right", etc. indicate orientation or positional relationships based on the orientation or positional relationships shown in the accompanying drawings, for ease of description only, and do not indicate or imply that the device or component referred to must have a specific orientation, be constructed and operated in a specific orientation, and therefore cannot be understood as limiting the patent. The terms "first" and "second" are only used for descriptive purposes and should not be understood as indicating or implying relative importance or implying the quantity of technical features. The meaning of "multiple/a plurality of" is two or more, unless otherwise specified.

It should be noted that in the embodiments of the present application, the same reference numerals are used to represent the same components or parts. For the same parts in the embodiments of the present application, only one part or component may be marked with a reference numeral in the figure. It should be understood that for other identical parts or components, the reference numerals are also applicable.

Hypertension is the most common chronic disease and a major risk factor for cardiovascular and cerebrovascular diseases, which can easily cause stroke, myocardial infarction, heart failure, and chronic kidney disease. Thus, it is known as the "invisible killer" that affects human health. Excessive excitation of the renal sympathetic nerves can cause blood pressure to rise, leading to a high risk of death and a heavy burden of treatment due to hypertension. RDN technology refers to the technique of destroying the renal sympathetic afferent and efferent nerves through interventional treatment methods (via the femoral and radial arteries), which can weaken the activity of the renal and systemic sympathetic nerves, thereby reducing blood pressure.

RDN technology may be based on manners such as radiofrequency ablation, ultrasound ablation, cryoablation, chemical ablation, and the like, but currently, radiofrequency ablation (rRDN) and ultrasound ablation (uRDN) are mainly dominant. Radiofrequency ablation is the process of delivering a catheter with electrodes to the renal artery and outputting radiofrequency energy through the electrodes, causing the surrounding tissue to heat up and gradually conducting radiofrequency current or heat below the renal artery adventitia. Although the temperature of electrodes and renal artery intima can be reduced by infusing cold saline during the radiofrequency ablation, there is still a risk of damaging the renal artery intima and causing renal artery stenosis.

Ultrasonic energy has the advantages of good penetration and high energy controllability, and thus has certain technical advantages in the field of RDN. At present, the existing technologies mainly include the schemes of circular transducers for 360 ° energy emission and planar transducers for directional energy emission. However, neither the circular transducers nor the planar transducers can achieve spatial distribution control of sound field and ultrasound energy, which can easily lead to the risk of damaging the renal artery vessel wall and causing renal artery stenosis, so it is necessary to cool the renal artery intima with cooling water.

Based on the above considerations, in order to alleviate the damage that ultrasound energy may cause to the blood vessel wall, this application provides an ablation catheter that includes an ultrasonic generator configured with a driving assembly and a generation assembly, wherein ultrasonic waves emitted by the generation assembly is able to converge to form a focal out of the blood vessel wall, thereby enabling the ultrasonic waves to have larger energy at the focal to ablate the sympathetic nerves at a position corresponding to the focal.

At positions where the ultrasonic waves pass through the blood vessel wall, the ultrasonic waves have not yet converged at the focal and thus are more dispersed and carry less energy, resulting in less heat generating of the blood vessel wall and thus alleviating the damage caused by the ultrasonic waves to the blood vessel wall.

This ablation catheter can not only ablate the target sympathetic nerves out of the blood vessel, but also alleviate the damage of the ultrasonic waves to the blood vessel wall. Moreover, this ablation catheter is simple in structure, which reduces the difficulty of operation and lowers the cost of the ablation catheter.

The ablation catheter provided in the embodiments of this application can be used not only for ablating the renal sympathetic nerves, but also for ablating other tissues, such as benign prostatic hyperplasia tissues. For the convenience of description, the following embodiments will take the ablation catheter provided in some embodiments of this application for ablating the renal sympathetic nerves as an example.

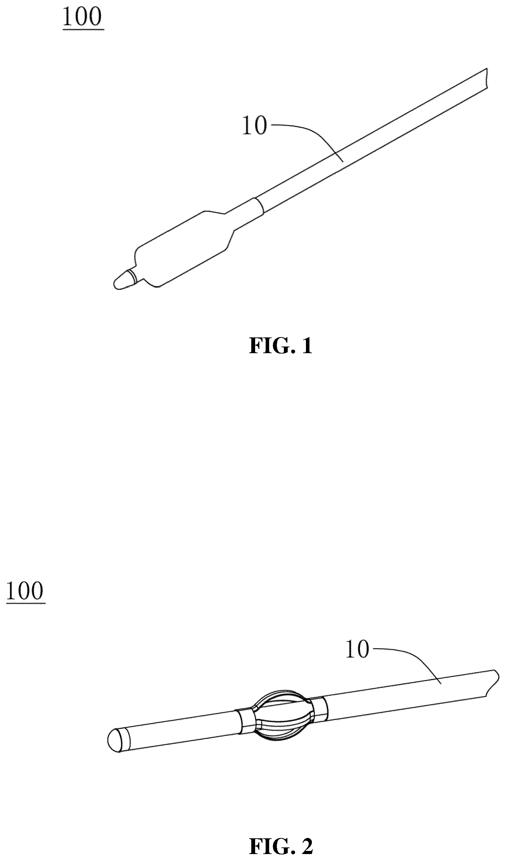

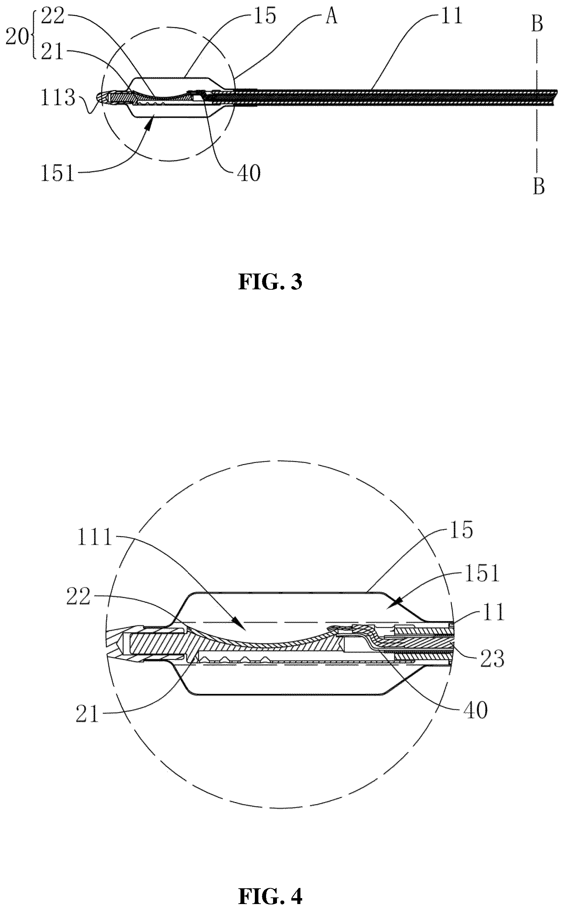

On one aspect, referring to FIG. 1 to FIG. 4, an embodiment of the present application provides an ablation catheter 100. Among them, FIG. 1 is a partial three-dimensional view of an ablation catheter 100 provided in an embodiment of the present application, FIG. 2 is a partial three-dimensional view of an ablation catheter 100 provided in another embodiment of the present application, FIG. 3 is a cross-sectional view of the ablation catheter 100 of FIG. 1, and FIG. 4 is an enlarged view of part A of FIG. 3. Because the ablation catheter 100 is generally elongated, FIG. 1 and FIG. 2 only show partial structures of end portions of the ablation catheter 100.

The ablation catheter 100 includes a catheter assembly 10 and an ultrasonic generator 20, wherein the ultrasonic generator 20 is arranged in the catheter assembly 10. The ultrasonic generator 20 includes a driving assembly 21 and a generation assembly 22 connected to the driving assembly 21. The driving assembly 21 is configured to drive the generation assembly 22 to vibrate, and the generation assembly 22 is configured to emit ultrasonic waves and converge the ultrasonic waves at a focus, wherein the focus is located out of the catheter assembly 10.

The catheter assembly 10 refers to a structure or structure assembly used to carry the ultrasonic generator 20 and other components. The catheter assembly 10 can enter and move along the patient's blood vessel. The ultrasonic generator 20 and other components are accommodated in the catheter assembly 10 and can move along with one end of the catheter assembly 10 to a target position. The catheter assembly 10 may include one or multiple tubular structures. The material of the catheter assembly 10 may be polytetrafluoroethylene, silicone rubber, polyurethane, etc. The shape of the catheter assembly 10 may be cylindrical, prismatic, etc.

The ultrasonic generator 20 refers to a structure or a structure assembly that can emit ultrasonic waves. The ultrasonic waves emitted by the ultrasonic generator 20 can penetrate the blood vessel wall and ablate the target sympathetic nerves. The ultrasonic generator 20 may include a transducer, various circuit boards, or other structures or components.

The driving assembly 21 refers to a structure or a structure assembly in the ultrasonic generator 20 used to drive the generation assembly 22 to vibrate. The driving assembly 21 may include various circuit boards, such as amplification circuit boards, and may further include structures such as matching layers, backing, etc. The driving assembly 21 is used to drive the generation assembly 22 to vibrate. For example, the driving assembly 21 may receive and process electrical signals, and send the processed electrical signals to the generation assembly 22.

The generation assembly 22 refers to a structure or a structure assembly in the ultrasonic generator 20 that can generate ultrasonic waves. The generation assembly 22 may only include one component capable of vibrating and generating ultrasonic waves, or include multiple components capable of vibrating and generating ultrasonic waves. The material of the generation assembly 22 may be barium titanate ceramic, milled lead titanate ceramic, etc. The generation assembly 22 is capable of receiving electrical signals sent by the driving assembly 21 and generating mechanical vibrations based on the received signals, thereby generating ultrasonic waves.

The ultrasonic waves generated by the generation assembly 22 can be converged at the focus, so that the ultrasonic waves generate a large amount of energy at the focus, which can be used to ablate the sympathetic nerves at a position corresponding to the focus. Specifically, the ultrasonic waves generated by the generation assembly 22 can be converged at the focus by setting a specific shaped component or setting multiple components. In some embodiments, the generation assembly 22 may include a component configured with specific shape. In this case, a specific shape of a piezoelectric sheet 221 may be arc-shaped, U-shaped, etc., so that the ultrasonic waves generated by the generation assembly 22 can be converged at the focus. In other embodiments, the generation assembly 22 may include multiple components which may be arranged along a trajectory being arc-shaped, U-shaped, L-shaped, etc., so that the ultrasonic waves generated by the generation assembly 22 can be converged at the focus. It may be understood that the generation assembly 22 may adopt other structures to converge the ultrasonic waves at the focus, not limited to the aforementioned manners.

Before the ultrasonic waves generated by generation assembly 22 converged at the focus, they were dispersed and carried less energy. Specifically, the ultrasonic waves closer to the ultrasound generator 20 carry less energy. Generally, an inner diameter of the blood vessel is smaller, and thus the ultrasonic waves are closer to the ultrasound generator 20 when they pass through the blood vessel wall, the energy carried by the ultrasonic waves is smaller, and the damage caused by the ultrasonic waves to the blood vessel wall is also smaller.

In this embodiment, the ultrasonic generator 20 is configured to include a driving assembly 21 and a generation assembly 22, and the ultrasonic waves emitted by the generation assembly 22 can be converged to form a focus at a position out of the blood vessel wall, thereby enabling the ultrasonic waves to have larger energy at the focus to ablate the sympathetic nerves at a position corresponding to the focus. Further, at positions where the ultrasonic waves pass through the blood vessel wall, the ultrasonic waves are more dispersed and carry less energy, resulting in less heat generating of the blood vessel wall and thus alleviating the damage of the ultrasonic waves to the blood vessel wall.

In some embodiments, the ultrasonic generator 20 is an ultrasonic transducer. In this case, the driving assembly 21 may include a flexible circuit board, a matching layer, a backing, etc. The generation assembly 22 may include a structure formed of piezoelectric material.

In some embodiments, the ultrasonic generator 20 is connected to an external equipment through a cable 23. One end of the cable 23 is connected to the driving assembly 21 or the generation assembly 22, and another end of the cable 23 can extend through the catheter assembly 10 and to the outside, facilitating the external equipment to send electrical signals to the ultrasonic generator 20 through the cable 23.

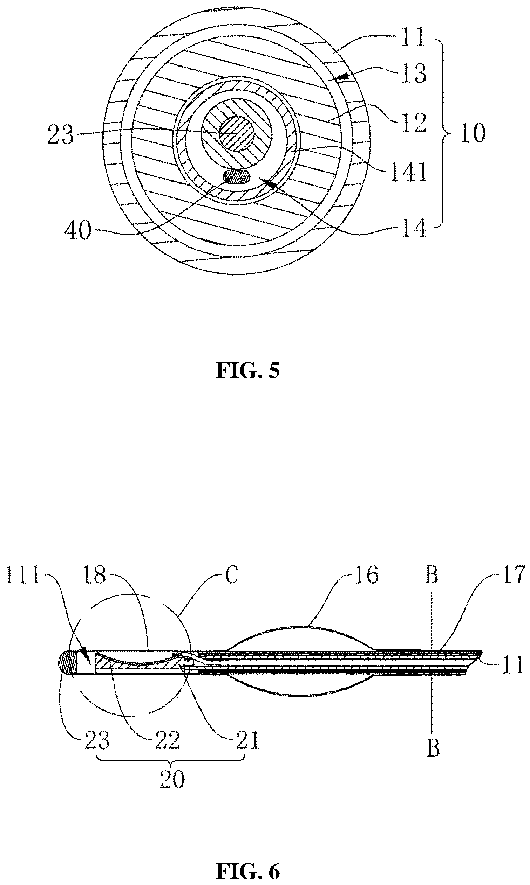

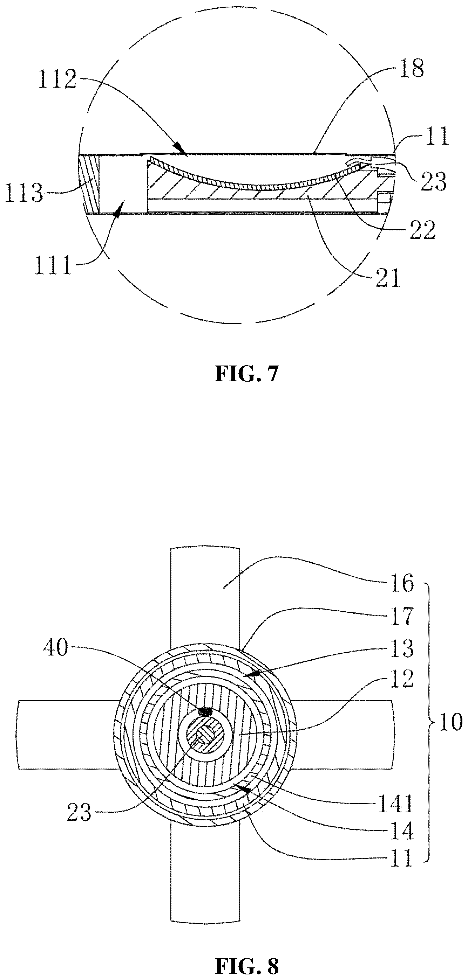

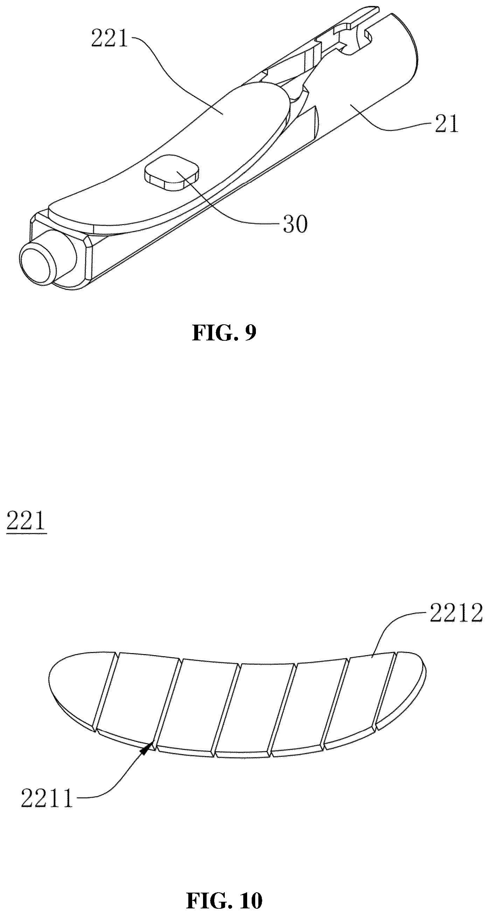

According to embodiments of the present application, reference may be made to FIG. 3, FIG. 4, FIG. 6, FIG. 7 and FIG. 9, wherein FIG. 3 is a cross-sectional view of an ablation catheter 100 provided in an embodiment of the present application, FIG. 4 is an enlarged view of part A of FIG. 3, FIG. 6 is a cross-sectional view of an ablation catheter 100 provided in another embodiment of the present application, FIG. 7 is an enlarged view of part C of FIG. 6, and FIG. 9 is a three-dimensional view of an ultrasonic generator 20 of an ablation catheter 100 provided in an embodiment of the present application.

The generation assembly 22 includes an arc-shaped piezoelectric sheet 221, and a circle center of the piezoelectric sheet 221, wherein the focus are disposed on the same side of the catheter assembly 10.

The piezoelectric sheet 221 refers to a structure that can receive electrical signals and generate mechanical vibrations. For example, the piezoelectric sheet 221 can receive electrical signals sent by the driving assembly 21 and generate internal stress inside the material, causing the material to vibrate and thus to form ultrasonic waves. The material of the piezoelectric sheet 221 may be barium titanate ceramic, milled lead titanate ceramic, etc.

The arc-shaped piezoelectric sheet 221 means that the shape of the piezoelectric sheet 221 is arc. Specifically, the shape of the piezoelectric sheet 221 may be configured as a part of a side wall of a cylindrical structure with thin wall, or a part of a wall of a spherical structure with thin wall, or other arc-shaped structures.

The circle center of the piezoelectric sheet 221 and the focus are set on the same side of the catheter assembly 10, that is, the piezoelectric sheet 221 is convex in a direction away from the focus.

When the arc-shaped piezoelectric sheet 221 receives electrical signals and vibrates, the generated ultrasonic waves can be converged at a position near the circle center or spherical center of the arc-shaped piezoelectric sheet 221, thereby achieving the effect of converging ultrasonic waves at the focus.

In this embodiment, the generation assembly 22 is configured to include the arc-shaped piezoelectric sheet 221. By emitting ultrasonic waves capable of being focused through the arc-shaped piezoelectric sheet 221, the ultrasonic waves can have larger energy at the focus, to achieve the effect of ablating the sympathetic nerves at a position corresponding to the focus.

In some embodiments, a radius of the piezoelectric sheet 221 in a range of 6mm to 15mm, specifically, the radius of the piezoelectric sheet 221 may be 6mm, 7mm, 8mm, 9mm, 10mm, 11mm, 12mm, 13mm, 14mm, 15mm, etc.

Since the ultrasonic waves generated by the piezoelectric sheet 221 can be converged at a position near the circle center or spherical center of the arc-shaped piezoelectric sheet 221, the position of the focus of the ultrasonic waves can be adjusted by adjusting the radius of the piezoelectric sheet 221.

Further, the sympathetic nerves are mainly located in the adipose tissue outside the renal artery adventitia, and a depth of the sympathetic nerves is in a range of 2mm~7mm from the renal artery intima. Therefore, the radius of the piezoelectric sheet 221 is set to be in a range of 6mm~15mm, so that the focus can be within the range of 2mm-7mm from the renal artery intima, thereby facilitating ablation of the sympathetic nerves at the focus through ultrasonic waves.

In this embodiment, the radius range of the piezoelectric sheet 221 is further limited, to limit the position range of the focus by limiting the radius range of the piezoelectric sheet 221, thus the ultrasonic waves can ablate sympathetic nerves at the focus and reduce damage to the vessel wall when the ultrasonic waves pass through the vessel wall.

In some embodiments, reference may be made to FIG. 10, which shows the specific structure of the piezoelectric sheet 221 provided in an embodiment of the present application.

At least one separating groove 2211 is defined in the piezoelectric sheet 221 to divide the piezoelectric sheet 221 into at least two piezoelectric sub-sheets 2212. The piezoelectric sub-sheets 2212 can rotate relative to the driving assembly 21.

The separating groove 2211 refers to a groove extending through the piezoelectric sheet 221. The separating groove 2211 can cut the piezoelectric sheet 221 to form two piezoelectric sub-sheets 2212. As the number of the separating grooves 2211 increases, the number of the piezoelectric sub-sheets 2212 will also increase. The separating groove 2211 may extend along the length direction of the piezoelectric sheet 221, or the width direction of the piezoelectric sheet 221. The separating groove 2211 may extend in other directions, and multiple separating grooves 2211 may be provided and extend in different directions.

The number of the piezoelectric sub-sheets 2212 may be two or more. In some embodiments, the number of the piezoelectric sub-sheets 2212 is an odd number. Furthermore, in the radial direction of the catheter assembly 10, the piezoelectric sub-sheets 2212 have odd columns, and the piezoelectric sub-sheets 2212 in each column are arranged along the length direction of the catheter assembly 10.

It can be understood that the multiple piezoelectric sub-sheets 2212 formed by the separating groove 2211 are all on the same arc-shaped surface, which can be a part of a cylindrical side wall, a part of a spherical wall, or other arc-shaped surfaces. This configuration enables the ultrasonic waves formed by the multiple piezoelectric sub-sheets 2212 to be converged at the circle center or spherical center of the arc-shaped surface.

The piezoelectric chip 2212 can rotate relative to the driving assembly 21. With the rotation of the piezoelectric chip 2212, the shape of the arc-shaped surface will change, and the position of the circle center or spherical center of the arc-shaped surface will also change. That is, the rotation of the piezoelectric chip 2212 can adjust the position of the focus, to facilitate the ablation of sympathetic nerves at different positions, thereby meeting different ablation needs.

The rotation of the piezoelectric chip 2212 relative to the driving assembly 21 may only in one direction, or in two or more directions. The rotation of the piezoelectric chip 2212 can be driven by a micro driving device or by other structures.

In this embodiment, the piezoelectric sheet 221 is divided into multiple piezoelectric sub-sheets 2212 through the separating groove 2211, and each piezoelectric sheet 2212 is movable relative to the driving assembly 21, thereby allowing each piezoelectric sheet 2212 to adjust its orientation and angle, and enabling the multiple piezoelectric sub-sheets 2212 to form a phased array structure, which facilitates the adjustment of the position of the focus by staff as needed and improves the compatibility of the ablation catheter 100.

In some embodiments, the separating groove 2211 may be filled with insulating and absorbing materials to reduce the influence between the piezoelectric sub-sheets 2212. The insulating and absorbing materials may be epoxy resin, silicone, etc.

According to some embodiments of the present application, the generation assembly 22 has a first state and a second state. An acoustic power of the ultrasonic waves emitted by the generation assembly 22 in the first state is greater than an acoustic power of the ultrasonic waves emitted by the generation assembly 22 in the second state, wherein the acoustic power of the ultrasonic waves emitted by the generation assembly 22 in the second state is less than or equal to 10% of that in the first state. For example, the acoustic power of the ultrasonic waves emitted by the generation assembly 22 in the second state may be 10%, 7%, 5%, 4%, 3%, 2%, 1%, etc. of that in the first state.

The acoustic power refers to the energy radiated outward per unit time by a sound source. The greater the acoustic power, the stronger the energy transmitted by the ultrasonic waves per unit time.

The second state of the generation assembly 22 is used for mapping the sympathetic nerves. As there are parasympathetic nerves near the sympathetic nerves, and the ablation of the parasympathetic nerves will have a negative impact on blood pressure control, the second state is thus set to distinguish the sympathetic nerves and parasympathetic nerves.

The first state of the generation assembly 22 is used for ablating of sympathetic nerve at the focus, so that the acoustic power of the ultrasonic waves emitted by the generation assembly 22 in the first state is greater than that in the second state, allowing the ultrasonic waves emitted by the generation assembly 22 in the first state to carry sufficient energy to ablate the sympathetic nerves.

Specifically, the acoustic power of the ultrasonic waves emitted by the generation assembly 22 in the second state is referred to as low power. The low-power ultrasonic waves carry less energy and will not cause tissue damage, but they can cause changes in nerve cell permeability and ion channels, resulting in neural stimulation reactions and changes in blood pressure. The stimulation reactions produced by sympathetic nerves and parasympathetic nerves have different effects on blood pressure. Therefore, the staff can distinguish the sympathetic nerves and parasympathetic nerves through different changes in blood pressure to achieve the mapping effect.

Because low-power ultrasonic waves do not cause damage to the sympathetic nerves, but can cause changes in pericardial permeability, ion channels, etc., and form neural stimulation responses, the staff can identify and determine the target nerves that need to be ablated through neural stimulation responses. Based on this, in this embodiment, the ultrasonic waves have two acoustic power ranges, so the ablation catheter 100 has both the ability to ablate nerves and the ability to identify nerves.

According to some embodiments of the present application, reference may be made to FIG. 10, which shows the specific structure of the piezoelectric sheet 221 provided in an embodiment of the present application.

The ablation catheter 100 further includes an imaging device 30, which is arranged within the catheter assembly 10 and located at a side of the ultrasonic generator 20.

The imaging device 30 refers to a device that can enter the blood vessel along with the catheter assembly 10 and obtain images near the blood vessel. The imaging device 30 can be an imaging ultrasound transducer, an optical coherence tomography (OCT) optical device, or other imaging devices.

The imaging device 30 may be communicated with and connected to external equipment to send the obtained images to the external equipment, making it easier for the staff to determine the position of the catheter assembly 10, the location of the ablation site, and other information based on the images, facilitating better ablation operations for the staff.

In some embodiments, the imaging device 30 is arranged at a side of the generation assembly 22 away from the driving assembly 21, and located in the direction of emission of the ultrasonic waves, so that the ultrasonic waves can reach the position where the imaging device 30 acquires the image information.

In this embodiment, the imaging device 30 is arranged next to the ultrasonic generator 20, so that the staff can obtain tissue images at corresponding positions to perform ablation operations.

According to some embodiments of the present application, reference may be made to FIG. 3, FIG. 5, FIG. 6, and FIG. 8, wherein, FIG. 3 is a cross-sectional view of an ablation catheter 100 provided by an embodiment of the present application, FIG. 5 is a cross-sectional view taken along line B-B of FIG. 3 and shows an internal structure of the catheter assembly 10, FIG. 6 is a cross-sectional view of the ablation catheter 100 provided by another embodiment of the present application, and FIG. 8 is a cross-sectional view taken along line D-D of FIG. 6 and shows an internal structure of the catheter assembly 10.

The catheter assembly 10 includes an outer catheter 11 and a rotary member 12 arranged inside the outer catheter 11. The outer catheter 11 is provided with a receiving chamber 111 therein, and the ultrasonic generator 20 is accommodated in the receiving chamber 111. One end of the rotary member 12 extends into the receiving chamber 111 and is connected to the ultrasonic generator 20. The rotary member 12 is bendable along with the outer catheter 11, and is rotatable relative to the outer catheter 11 to drive the ultrasonic generator 20 to rotate.

The outer catheter 11 refers to a structure or a structure assembly used to carry the rotary member 12, the ultrasonic generators 20, etc. The material of the outer catheter 11 may be polytetrafluoroethylene, silicone rubber, polyurethane, etc. The shape of the outer catheter 11 may be cylindrical, prismatic, etc.

The receiving chamber 111 refers to a space inside the outer catheter 11. The receiving chamber 111 may only refer to the space set inside the outer catheter 11, or may be a space enclosed by a structure inside the outer catheter 11. The receiving chamber 111 is mainly used to accommodate the ultrasonic generator 20. The receiving chamber 111 may also accommodate the imaging device 30 or other structures and devices therein.

The rotary member 12 refers to a structure that is bendable and still rotatable after bending. The rotary member 12 has good torque transmission performance and flexibility. The rotary member 12 may be a torque spring tube or any other structure that is bendable and still rotatable after bending.

The ultrasonic generator 20 is accommodated in the receiving chamber 111. When the outer catheter 11 moves in the blood vessel, it can drive the ultrasonic generator 20 to move synchronously to reach the target position.

The rotary member 12 is accommodated inside the outer catheter 11. The rotary member 12 may be coaxially arranged with the outer catheter 11 or eccentrically arranged without being coaxial. The outer catheter 11 can drive the rotary member 12 to move synchronously when it moves inside the blood vessel. One end of the rotary member 12 can extend into the receiving chamber 111 and be connected to the ultrasonic generator 20, so that the rotary member 12 can drive the ultrasonic generator 20 to rotate inside the receiving chamber 111. It can be understood that the other end of the rotary member 12, which is far away from the ultrasonic generator 20, can extend to the outside, so that the staff can rotate the rotary member 12 through equipment or manually.

The rotary member 12 can bend synchronously along with the outer catheter 11, and at the same time, after the bending of the outer catheter 11, the rotary member 12 can still drive the ultrasonic generator 20 to rotate inside the receiving chamber 111. Because blood vessels in vascular patients are usually tortuous, the ultrasonic generator 20 usually undergoes multiple bend positions before reaching the target position. Therefore, using the rotary member 12 to drive the ultrasonic generator 20 can better drive ultrasonic generator 20 to rotate inside the receiving chamber 111.

The rotary member 12 drives the ultrasonic generator 20 to rotate, allowing the ablation catheter 100 to better adjust the ablation position according to the actual situation of the patient, increasing the applicability of the ablation catheter 100. Because the sympathetic nerves of the renal artery mainly wrap around the renal artery in a network structure, the rotary member 12 drives the ultrasonic generator 20 to rotate, so that the focus of the ultrasonic waves can also rotate approximately around the blood vessel, forming a circular ablation area around the blood vessel, facilitating ablation of the sympathetic nerves around the blood vessel, thereby improving the ablation efficiency and reducing the situation of the ablation catheter 100 reciprocating into and out of the blood vessel.

In this embodiment, the catheter assembly 10 is configured to include a rotary member 12, which drives the ultrasonic generator 20 to rotate. The rotary member 12 can bend along with the outer catheter 11 when the outer catheter 11 bends, and the rotary member 12 can also drive the ultrasonic generator 20 to rotate when the outer catheter 11 bends, so that the focus of the ultrasonic waves generated by the ultrasonic generator 20 can move around the blood vessel, increasing the ablation area and better adapting to the distribution of sympathetic nerves of the renal artery.

In some embodiments, one end of the outer catheter 11 is provided with an end fitting 113, which can seal the end of the outer catheter 11, thereby reducing the occurrence of liquid or other substances entering the blood vessel from the outer catheter 11 or the receiving chamber 111, and also reducing the occurrence of substances entering the outer catheter 11 from the blood vessel.

The end fitting 113 further has the function of guiding the movement of the ablation catheter 100 inside the blood vessel, so that an end of the end fitting 113 away from the outer catheter 11 may be configured as a pointed end to reduce the moving resistance of the ablation catheter 100 inside the blood vessel. The end of the end fitting 113 away from the outer catheter 11 may be configured with a round head, which can reduce the moving resistance of the ablation catheter 100 the blood vessel and also minimize the damage of the end fitting 113 to the blood vessel wall.

In some embodiments, the catheter assembly 10 further includes an inlet channel 13 and an outlet channel 14 provided on the outer catheter 11, wherein one end of each of the inlet channel 13 and the outlet channel 14 is communicated with the receiving chamber 111.

The inlet channel 13 and the outlet channel 14 both refer to channel structures or structural assemblies for transporting liquids. One end of the inlet channel 13 and one end of the outlet channel 14 are both communicated with the receiving chamber 111. The inlet channel 13 is used to deliver liquid into the receiving chamber 111, and the outlet channel 14 is used to discharge the liquid in the receiving chamber 111, thereby forming a circulating flow to reduce the temperature inside the receiving chamber 111.

The liquid flowing in the inlet channel 13 and the outlet channel 14 is used to lower the temperature inside the receiving chamber 111. The liquid may be water, ethylene glycol, etc.

Due to the heat generating of the ultrasonic generator 20 during its operation, and the ultrasonic waves themself carrying heat, in order to reduce the damage to the blood vessel wall caused by excessive heat, the inlet channel 13 and outlet channel 14 are set up to form a circulating flow through the receiving chamber 111, thereby reducing the temperature inside the receiving chamber 111 and minimizing the possible damage to the blood vessel wall caused by high temperature.

It can be understood that both the inlet channel 13 and the outlet channel 14 can be connected to the outside at the ends thereof away from the receiving chamber 111, so that external equipment can deliver liquid to the receiving chamber 111 through the inlet channel 13, and the liquid in the receiving chamber 111 can be discharged to the outside through the outlet channel 14.

The inlet channel 13 and the outlet channel 14 can be arranged inside or outside the outer catheter 11. In some embodiments, an inlet pipe and an outlet pipe are respectively provided outside the outer pipe 11, and the inlet channel 13 and the outlet channel 14 are formed inside the inlet pipe and the outlet pipe, respectively. In other embodiments, an inlet pipe and an outlet pipe are provided inside the outer catheter 11, and the inlet channel 13 and the outlet channel 14 are formed inside the inlet pipe and the outlet pipe, respectively. In some embodiments, a partition structure is provided inside the outer catheter 11, which cooperates with a catheter wall of the outer catheter 11 to enclose the inlet channel 13 and the outlet channel 14.

In some embodiments, a water pipe 141 is provided inside the outer catheter 11. The inlet channel 13 is formed between an outer wall of the water pipe 141 and an inner wall of the outer catheter 11, while the outlet channel 14 is formed inside the inner wall of the water pipe 141.

The cable 23 may be installed inside the water pipe 141 or between the outer wall of water pipe 141 and the inner wall of the outer catheter 11. The rotary member 12 can be coaxially arranged with the water pipe 141 and mounted around the water pipe 141. The rotary member 12 can also be coaxially arranged with the water pipe 141 and inserted into the water pipe 141. The rotary member 12 may not be coaxially arranged with the water pipe 141.

In this embodiment, an inlet channel 13 and an outlet channel 14 are provided on the catheter assembly 10 and communicated with the receiving chamber 111, thereby forming a circulating water flow inside the catheter assembly 10. The circulating water flow is used to cool and reduce the temperature of the ultrasound generator 20, thereby reducing the damage to the blood vessel wall caused by the high temperature of the ultrasound generator 20.

In some embodiments, the ablation catheter 100 further includes a temperature sensor 40 arranged at a side of the ultrasonic generator 20.

The temperature sensor 40 refers to a component used to detect the temperature near the ultrasonic generator 20. The temperature sensor 40 can be communicated with the external equipment to send real-time temperature information to the outside. The staff can operate based on the real-time temperature information, such as stopping the device, increasing the liquid flow rate, increasing the liquid flow velocity, etc., when the real-time temperature is high.

The temperature sensor 40 can be connected to external equipment through a signal wire in the outer catheter 11, or wirelessly connected to external equipment, or connected to external equipment through other communication methods. In some embodiments, the temperature sensor 40 is a cable-shaped thermocouple temperature sensor that is installed in the outer catheter 11 with one end thereof extending to a lateral side of the ultrasonic generator 20.

In this embodiment, a temperature sensor 40 is installed at a side of the ultrasound generator 20 to facilitate the monitoring of the temperature of the ultrasound generator 20 by the staff, thereby reducing the potential damage to the blood vessel wall caused by high temperature.

In some embodiments, the ultrasonic generator 20 may further include a base or a housing for providing a fixed foundation for the driving assembly 21, the generation assembly 22, and the cable 23. In this case, one end of the temperature sensor 40 may be accommodated inside the base or the housing and located at a position close to the piezoelectric plate 221, to better obtain real-time temperature information near the piezoelectric plate 221.

According to some embodiments of the present application, reference may be made to FIG. 3 and FIG. 4, wherein FIG. 3 is a cross-sectional view of an ablation catheter 100 provided by an embodiment of the present application, and FIG. 4 is an enlarged view of part A of FIG. 3, showing the specific structure inside a covering cavity 151.

The catheter assembly 10 further includes a covering film 15 arranged around the outer catheter 11. The covering film 15 forms the covering cavity 151 around an outer periphery of the outer catheter 11. The covering cavity 151 is communicated with the receiving cavity 111. The covering film 15 can move in a direction towards or away from the receiving cavity 111.

The covering film 15 refers to a thin, film-like structure or structure assembly set around the outer catheter 11, and can encircle a covering cavity 151 outside the outer catheter 11. The covering cavity 151 is a sealed structure and can not communicate with the outside.

The covering cavity 151 is communicated with the receiving chamber 111, that is, the covering cavity 151 covers the receiving chamber 111 outside the outer catheter 11. In some embodiments, the outer catheter 11 is divided into two sections, i.e., front and rear sections, by the receiving chamber 111. A space between the front and rear sections is the receiving chamber 111, which is an open space set between the front and rear sections. The covering cavity 151 covers and communicates with the receiving chamber 111, that is, the receiving chamber 111 is located inside the covering cavity 151 and is part of the space of the covering cavity 151. In other embodiments, a through-hole or passage is defined in the outer catheter 11, and the receiving chamber 111 and the covering cavity 151 are communicated with each other through the through-hole or passage.

Due to the covering cavity 151 enclosing the receiving chamber 111, and the ultrasonic generator 20 being accommodated in the receiving chamber 111, the covering film 15 is designed as a thin film structure to reduce the negative impact of the covering film 15 on the ultrasonic waves, such as minimizing energy attenuation during the transmission of the ultrasonic waves through the covering film 15. The material of the covering film 15 may be silicone rubber, polyurethane (PU), polytetrafluoroethylene (PIFE), polyamide (PA), polyethylene terephthalate (PET), etc. The thickness of the covering film 15 should be relatively thin to reduce the negative impact on the ultrasonic waves, while also should have a certain strength. For example, the thickness of the covering film 15 may be 0.01mm, 0.02mm, 0.03mm, etc.

When the outer catheter 11, along with the ultrasonic generator 20, enter the patient's blood vessel and reach the target position, liquid, through the inlet channel 13, can enter the receiving chamber 111 and the covering cavity 151. At this time, the outlet channel 14 can be closed first. As the liquid enters, the covering cavity 151 can expand gradually. In this case, the covering film 15 moves away from the receiving chamber 111 until it contacts the blood vessel wall, achieving the effect of fixing the outer catheter 11, which in turn fixes the position of the ultrasonic generator 20, thereby reducing the possible shaking of the ultrasonic generator 20 during use. After the ablation is completed, the liquid is no longer delivered into the receiving chamber 111 through the inlet channel 13, and the liquid in the covering cavity 151 can be discharged through the outlet channel 14. At this time, the covering film 15 gradually moves towards the receiving chamber 111 and separates from the blood vessel wall as the liquid decreases, to facilitate the removal of the outer catheter 11 and reduce the negative impact that may be caused by the removal of the outer catheter 11. In some embodiments, when there is no liquid in the covering cavity 151, the covering film 15 may be attached to the outer catheter 11 to better reduce the negative effects that may occur during the removal of the outer catheter 11.

In this embodiment, a covering film 15 is set outside the catheter 11, and a covering cavity 151 is enclosed by the covering film 15 and communicated with the receiving cavity 111. Thus, water can enter the covering cavity 151 after entering the receiving cavity 111. The covering cavity 151 can gradually expand with the inflow of water, and thus the covering film 15 can move away from the receiving cavity 111 to abut the blood vessel wall, thereby fixing the catheter assembly 10.

According to some embodiments of the present application, reference may be made to FIG. 2, FIG. 6, and FIG. 7, wherein FIG. 2 is a partial three-dimensional view of an ablation catheter 100 provided by an embodiment of the present application, FIG. 6 is a cross-sectional view of the ablation catheter 100 of FIG. 2, and FIG. 7 is an enlarged view of part C of FIG. 6, showing the specific structure inside the covering cavity 151.

The catheter assembly 10 further includes a support member 16 and a moving member 17 arranged outside the outer catheter 11. One end of the support member 16 is connected to the outer catheter 11, and another end of the support member 16 is connected to the moving member 17;

The moving member 17 is connected to the outer catheter 11 in a movable manner, and can move relative to the outer catheter 11 along an axial direction of the outer catheter 11, and can drive the support component 16 to deform in a direction away from or close to the outer catheter 11.

The support member 16 refers to a support structure or structure assembly located outside the outer catheter 11. The shape of the support member 16 may be strip-like, sheet-like, etc. The material of the support member 16 may be metal, plastic, composite material, etc.

The moving member 17 refers to a structure or structure assembly that can move along the axial direction of the outer catheter 11. The shape of the moving member 17 may be cylindrical-shaped, sheet-like, strip-shaped, etc. The material of the moving member 17 may be metal, plastic, composite material, etc. In some embodiments, the moving member 17 is a sleeve being mounted around and coaxial with the outer catheter 11, and the moving member 17 is bendable and deformable.

The support member 16 can be attached to the outer catheter 11 and is deformable in a direction away from the outer catheter 11. When the support member 16 deforms in the direction away from the outer catheter 11, it can come into contact with the blood vessel wall to fix the position of the outer catheter 11, thereby fixing the ultrasonic generator 20.

One end of the support member 16 is connected to the outer wall of the outer catheter 11, and the other end of the support member 16 is connected to the moving member 17. When the moving member 17 moves towards the support member 16 along the axial direction of the outer catheter 11, the middle portion of the support member 16 is compressed and deformed in the direction away from the outer catheter 11. When the moving member 17 moves away from the support component 16 along the axial direction of the outer catheter 11, the middle portion of the support component 16 is subjected to tensile deformation and deforms towards the direction closer to the outer catheter 11 to contact the outer wall of the outer catheter 11.

In this embodiment, a support member 16 and a moving member 17 are provided on the outer catheter 11. The support member 16 can deform in a direction away from the outer catheter 11 along with the movement of the moving member 17 to abut the blood vessel wall, thereby fixing the position of the outer catheter 11. The support member 16 can also deform towards the outer catheter 11 to facilitate the entry of the catheter assembly 10 into the blood vessel and its movement within the vessel.

In some embodiments, the support member 16 can be configured in an arc shape and is convex in the direction away from the outer catheter 11, so that the support member 16 can deform in the direction away from the outer catheter 11.

In some embodiments, the support member 16 is a sheet-like structure, and there are multiple support members 16. The multiple support members 16 are evenly spaced on the circumferential side of the outer catheter 11. When the moving member 17 moves in the direction close to the support member 16 and makes the support members 16 deform, the multiple support members 16 deform to form a lantern-shaped structure.

In some embodiments, to reduce the negative impact of the support members 16 on the ultrasonic waves generated by the ultrasonic generator 20, the support members 16 and the ultrasonic generator 20 are arranged in a staggered manner along the axial direction of the outer catheter 11.

According to some embodiments of the application, reference may be made to FIG. 6 and FIG. 7, wherein FIG. 6 is a cross-sectional view of the ablation catheter 100 provided in an embodiment of the present application, and FIG. 7 is an enlarged view of part C of FIG. 6, showing the specific structure inside the covering cavity 151.

The outer catheter 11 is provided with an opening 112 that is communicated with the receiving chamber 111, and the opening 112 faces towards the ultrasonic generator 20. There is a transaudient film 18 set on the opening 112, which is used to seal the opening 112 and allow the ultrasonic waves to pass through.

The opening 112 is defined in the outer catheter 11, and the receiving chamber 111 can communicate with the outside through the opening 112. The transaudient film 18 is provided on the outer catheter 11 and seals the opening 112, so that the liquid in the receiving chamber 111 cannot flow out of the outer catheter 11 through the opening 112. At the same time, ultrasonic waves can pass through the transaudient film 18, and substances from the blood vessel or the outside cannot enter the receiving chamber 111 through the transaudient film 18.

Compared to the convergence of ultrasonic waves through the catheter wall of the outer catheter 11 towards the target position, the transaudient film 18 can reduce the negative impact on the ultrasonic waves, such as reducing the attenuation of ultrasonic energy during transmission of the ultrasonic waves. The thickness of the transparent film 18 should be relatively thin to reduce the negative impact on the ultrasonic waves, while also having a certain strength. For example, the material of the transparent film 18 may be silicone rubber, PU, PIFE, PA, PET, etc. The thickness of the transparent film 18 may be 0.01mm, 0.02mm, 0.03mm, or other thicknesses.

Based on the function of the transaudient film 18, the opening 112 faces towards the ultrasonic generator 20, this enables the ultrasonic waves to be transmitted through the transaudient film 18 to the target position.

In this embodiment, an opening 112 is provided on the outer catheter 11 and faces towards the ultrasonic generator 20, and a transparent film 18 is installed on the opening 112 to reduce the influence of the catheter wall of the outer catheter 11 on the ultrasound transmission. At the same time, the sealing of the receiving chamber 111 can be maintained through the transparent film 18, to prevent water flow or other impurities from entering the blood vessel.

According to some embodiments of the present application, the ablation catheter 100 can be used not only to ablate sympathetic nerves near the renal artery, but also to ablate benign prostatic hyperplasia tissues. This embodiment takes the ablation of benign prostatic hyperplasia tissues as an example for further explanation, the ablation catheter 100 can not only ablate benign prostatic hyperplasia tissues at the target position, but also reduce damage to the urethral wall.

Since the ablation catheter 100 can enter from the urethra during the ablation process of benign prostatic hyperplasia tissues, the length of the ablation catheter 100 thus can be relatively short, that is, the lengths of the outer catheter 11, the rotary member 12, the inlet channel 13, and the outlet channel 14 can all be relatively short.

Because during the ablation process of benign prostatic hyperplasia tissues, the target position that needs to be ablated may be far away from the urethra, with the farthest point reaching 1cm away from the urethra. In this case, the radius of the piezoelectric film 221 should be larger, such as 13mm, 14mm, 15mm, etc., so that the focus can reach a position of about 1cm away from the urethra, allowing the ultrasonic waves to ablate benign prostatic hyperplasia tissues at the focus.

Due to the structural characteristics of the urethra, specifically, because the urethra is mostly straight and not curved, the ablation catheter 100 may be un-bendable, that is, the outer catheter 11 may be un-bendable. At this time, the rotary member 12 may also not have the ability to bend and can only rotate inside the outer catheter.

Due to the structural characteristics of the urethra, specifically, the width of the urethral opening and the inner diameter of the urethra are generally the same. Therefore, the ablation catheter 100 can be positioned directly by controlling the outer diameter of the catheter assembly 10 without the need for positioning structures such as the covering film 15 or the support member 16.

On the other hand, embodiments of the present application further provide an ablation device including the ablation catheter 100 provided in the above embodiments, so that the ablation device can ablate the sympathetic nerves at the target position and reduce damage to the blood vessel wall during the ablation process.

The ablation device may further include a structure or structure assembly such as a handle, a host, etc. For example, the host can provide electrical signals to the ultrasonic generator 20, receive signals from the temperature sensor 40 and the imaging device 30, provide liquid to the inlet channel 13, and receive liquid discharged from the outlet channel 14. For example, the handle may be held by the staff, and used to control the conductivity of the inlet channel 13 and the outlet channel 14, as well as the movement of the moving member 17.

The above embodiments are only used to illustrate the technical solutions of the present application, and not to limit the present application. Although the present application has been described in detail with reference to the foregoing embodiments, for those skilled in the art, the technical solutions described in the foregoing embodiments can still be modified, or some of the technical features can be equally replaced. Any modifications and equivalent replacements made within the spirit and principle of the present application should be within the scope of the present application.

## Claims

1. An ablation catheter, comprising:
a catheter assembly; and
an ultrasonic generator arranged in the catheter assembly, wherein the ultrasonic generator comprises a driving assembly and a generation assembly connected to the driving assembly, the driving assembly is configured for driving the generation assembly to vibrate, and the generation assembly is configured for emitting ultrasonic waves and converging the ultrasonic waves to a focus, wherein the focus is located out of the catheter assembly.

2. The ablation catheter according to claim 1, wherein the generation assembly comprises an arc-shaped piezoelectric sheet, and wherein a circle center of the piezoelectric sheet and the focus are disposed on the same side of the catheter assembly.

3. The ablation catheter according to claim 2, wherein a radius of the piezoelectric sheet is in a range of 6mm to 15mm.

4. The ablation catheter according to claim 2, wherein at least one separating groove is defined in the piezoelectric sheet to divide the piezoelectric sheet into at least two piezoelectric sub-sheets, and the piezoelectric sub-sheets are rotatable relative to the driving assembly.

5. The ablation catheter according to claim 2, wherein there is no separating groove defined in the piezoelectric sheet.

6. The ablation catheter according to any one of claims 1-4, wherein the generation assembly operates in a first state or a second state, and an acoustic power of the ultrasonic waves emitted by the generation assembly in the second state is less than or equal to 10% of that in the first state.

7. The ablation catheter according to any one of claims 1-4, wherein the ablation catheter further comprises an imaging device arranged in the catheter assembly and located at a lateral side of the ultrasonic generator.

8. The ablation catheter according to any one of claims 1-4, wherein the catheter assembly further comprises an outer catheter and a rotary member arranged in the outer catheter, the outer catheter is provided with a receiving chamber, and the ultrasonic generator is accommodated in the receiving chamber; and
one end of the rotary member extends into the receiving chamber and is connected to the ultrasonic generator, and the rotary member is bendable along with the outer catheter and rotatable relative to the outer catheter to drive the ultrasonic generator to rotate.

9. The ablation catheter according to claim 8, wherein the catheter assembly further comprises an inlet channel and an outlet channel provided on the outer catheter, and one end of the inlet channel and one end of the outlet channel both are communicated with the receiving chamber.

10. The ablation catheter according to claim 9, wherein the catheter assembly further comprises a covering film arranged around the outer catheter, the covering film encircles a covering cavity around an outer periphery of the outer catheter, the covering cavity is communicated with the receiving chamber, and the covering film is movable in a direction towards or away from the receiving chamber.

11. The ablation catheter according to claim 8, wherein the catheter assembly further comprises a support member and a moving member arranged out of the outer catheter, one end of the support member is connected to the outer catheter, and another end of the support member is connected to the moving member; and
the moving member is connected to the outer catheter in a movable manner, the moving member is movable relative to the outer catheter along an axial direction of the outer catheter, and is able to drive the support member to deform in a direction away from or close to the outer catheter.

12. The ablation catheter according to claim 8, wherein an opening is defined in the outer catheter and communicates with the receiving chamber, and the opening is oriented toward the ultrasonic generator; and
a transaudient film is provided on the opening, wherein the transaudient film is configured to seal the opening and allow ultrasonic waves to pass through.

13. The ablation catheter according to any one of claims 1-4, wherein the ablation catheter further comprises a temperature sensor arranged at a lateral side of the ultrasonic generator.

14. An ablation device, comprising the ablation catheter according to any one of claims 1-13.
